# EUROPEAN PATENT SPECIFICATION

(11) **EP 2 022 450 B1**
(45) Date of publication and mention of the grant of the patent: **15.06.2011**
(21) Application number: 07425486.3
(22) Date of filing: 31.07.2007
(51) Int. Cl.: A61F 5/37

(54) **Antirotation orthopedic cushion, in particular for post-traumatic and post-surgical treatment of the shoulder**
Orthopädisches Kissen mit Drehschutz, im Besonderen für posttraumatische und postchirurgische Behandlung der Schulter
Coussin orthopédique anti-rotation, en particulier pour le traitement post-traumatique et post-chirurgical de l'épaule

(43) Date of publication of application: 11.02.2009
(73) Proprietor: F.G.P. Srl, 37062 Dossobuono (VR) (IT)
(72) Inventor: Ferrigolo, Moreno, 37062 Dossobuono (VR) (IT); Turrini, Alberto, 37062 Castel D'Azzano (VR) (IT)
(74) Representative: Sandri, Sandro

(56) References cited:
- WO-A-03/071994
- FR-A- 2 771 625
- US-A1- 2005 273 026

## Description

### TECHNICAL FIELD

This invention concerns an antirotation orthopedic cushion, in particular for the post-traumatic and post-surgical treatment of the shoulder.

More specifically, this invention refers to an orthopedic cushion or orthosis designed to act as a useful support in the post-traumatic and post-surgical treatment of the shoulder, its main feature being to prevent the retroposition of the scapula-thoracic joint, making it possible at the same time to maintain this joint in a position corresponding to a constant abduction of around 15° and to be able to block it at various degrees of extrarotation and intrarotation.

This cushion substantially consists of three main elements: a cushion, a wedge and a "restraining" system.

This invention can be applied in the industrial sector for the production of orthopedic equipment and in particular in the sector of equipment for maintaining the arm joints and specifically the rotator cuffs in a state of immobilisation.

### BACKGROUND ART

It is known that after surgical operations on the arm and the shoulders, particularly after operations for reconstruction of the rotator cuffs, it is necessary for the patient to wear auxiliary orthopedic devices or orthoses, that is to say fixed or removable devices that make it possible to keep the arm in as stable a position as possible for a certain rehabilitation period.

After an operation for reconstruction of the rotator cuffs, the arm is normally maintained in a blocked position, first totally and then partially, by means of special orthopedic devices which are designed to immobilise the joint, by means of structures that allow the arm to be held against the body.

These orthopedic devices consist in some cases of actual adjustable angle mechanical joints, while in other cases pads or cushions are used.

In both cases, these orthopedic devices are positioned under the arm and are fixed, by special fastenings, securing the arm against the patient's waist or body.

Mechanical joints present considerable disadvantages both from the point of view of their construction complexity and as regards their dimensions and weight which are excessive.

To overcome the problems of mechanical equipment, the background art includes solutions that foresee the use of orthopedic abductor cushions, which consist of a soft material body of a certain volume and a certain shape, which is fixed by means of straps to the patient's side, remaining in place blocked under the arm in such a way that the arm can rest on it.

In this case, however, the problem that was encountered concerns the difficulty in producing abductor cushions that are able to immobilise the arm at the angle required by the orthopedic surgeon according to the particular case.

Orthopedic cushions with a variable configuration have therefore been produced, that is to say with shapes that allow them to be positioned in two distinct angular ways, generally with the possibility of two different angles between the arm and the body, for example 15 and 45 degrees.

This is achieved by the particular shape of the cushion, which if positioned in one way keeps the arm blocked at a certain angle, and if positioned on the its other side keeps the arm blocked at a different angle.

Problems have been encountered in this case too, mainly concerning the impossibility of immobilising the arm which has undergone reconstruction of the rotator cuffs, it being extremely difficult to position the arm at intermediate, greater or lesser angles than those indicated. The patient or the orthopedic surgeon is therefore often forced to resort to empirical solutions in order to position the cushion at the required angle.

This applicant has also patented another invention in patent VR2004A000130 concerning an orthopedic abductor cushion characterised in that it is inflatable and consists of a two-part casing. The first part, facing the inside, consists of a layer in pneumatic inflatable material, while the second part, facing the outside, consists of a layer made from a particular "Velcro-fixable" material.

Although it solves the problem of the complexity and size of the known cushions, this solution too was not satisfactory from the versatility point of view, since it could not for example prevent the retroposition of the scapula-thoracic joint. Nor was it able to prevent the problems encountered in the post-operative period, for example in cases where it is necessary to protect newly formed structures or stitches, etc.

This is due to the fact that in traditional devices the orthosis is designed to block the joint in just a few positions, for example at 15° abduction and 15° extrarotation or at 15° abduction and 30° extrarotation. It was not possible to foresee intermediate or greater angles than these ones.

Another problem with known solutions concerns the traditional strap-fastening systems, which are quite complex and cumbersome, considerably decreasing the level of comfort and making it very difficult to better adapt the system to the anatomical shape of the patient.

Document WO 03/071994 discloses a variable support of the arm or the shoulder which may be used for the conservative or postoperative treatment of vaious injuries of the shoulder. The support comprise a distal element, a proximal element and an axillary wedge that can be adjusted relative to the distal element and or the proximal element, at least in height.

Document FR-A-2771625 discloses an orthosis comprising a supporting block having a torso-shaped face and a supporting area for the forearm, as well as a strap for fixing the orthosis to the torso of the patient, and another strap for keeping the forearm on the supporting area.

Document US 2005/0273026 A1 discloses a shoulder stabilizing element including a pillow and a forearm wrap. The pillow has an arcuately-configured posterior face for engaging an anterior quadrant of a torso on a first side of a user. Furthermore, the pillow has a flat forearm engagement face for engaging a forearm on the first side of the user.

### DESCRIPTION OF THE INVENTION

This invention proposes to provide an antirotation orthopedic cushion, in particular for the post- traumatic and post-surgical treatment of the shoulder, which can be used in the post-operative period of reconstruction of the rotator cuffs. The particularity of this device is the presence of a surface designed to prevent the retroposition of the scapula-thoracic joint, making it impossible for the wearer to perform this movement and thus preventing the problems in the post-operative period, for example the need to protect newly constructed structures or stitches, etc..

The solution in question is thus able to eliminate or at least reduce the problems described above.

The adoption of a solution according to the invention is also useful when the patient has to wear the device at night when the state of not being awake implies that movements are less controlled.

The invention also proposes to provide an antirotation orthopedic cushion in particular for the post-traumatic and post-surgical treatment of the shoulder that is easy to produce in order to be economically advantageous.

This is achieved by means of an antirotation orthopedic cushion with the features described in the main claim.

The dependent claims describe advantageous embodiments of the invention.

The antirotation orthopedic cushion in particular for the post-traumatic and post-surgical treatment of the shoulder according to the invention therefore foresees the use of two components: the actual cushion which is a foam structure that comes into contact with the patient's arm, and a wedge which is positioned between the cushion and the patient's chest. These components are joined together by means of Velcro.

The wedge has a dual function; on one hand it ensures the possibility of adapting the orthosis to different anatomical shapes, thus reducing production costs (one size fits all) without this affecting the functionality of the product, and on the other it has a therapeutic function, making it possible for the health care operator to block the patient's joint not only in a neutral position but also in extrarotation or intrarotation.

### DESCRIPTION OF THE DRAWINGS

Other features and advantages of the invention will become evident on reading the description given below of one embodiment of the invention, given as a non-binding example, with the help of the accompanying drawings in which:

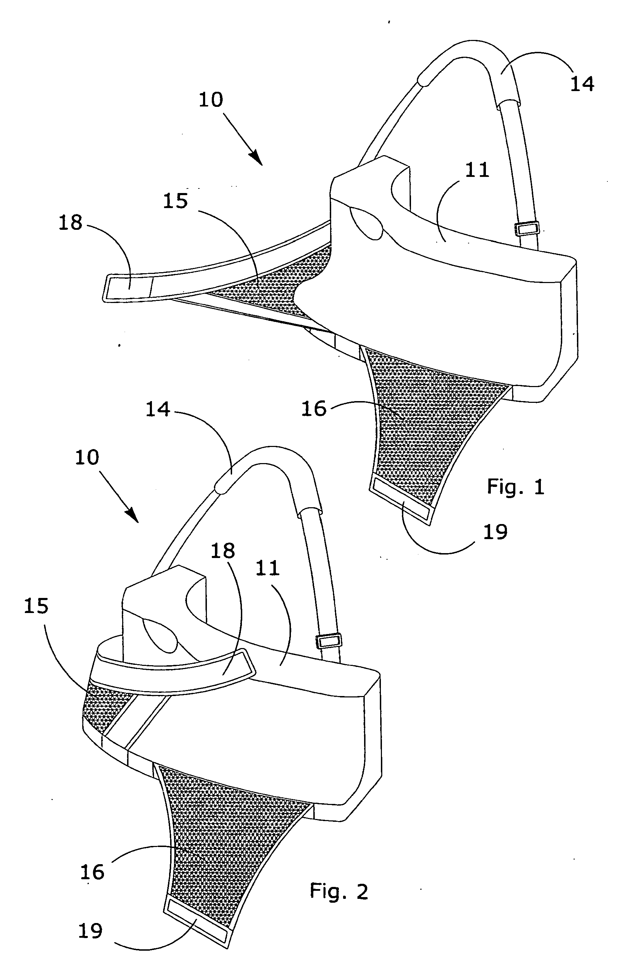

- figure 1 is a schematic view of the antirotation orthopedic cushion according to the invention in the open position;
- figure 2 is a schematic view of the antirotation orthopedic cushion according to the invention with a first fastening flap in the closed position;

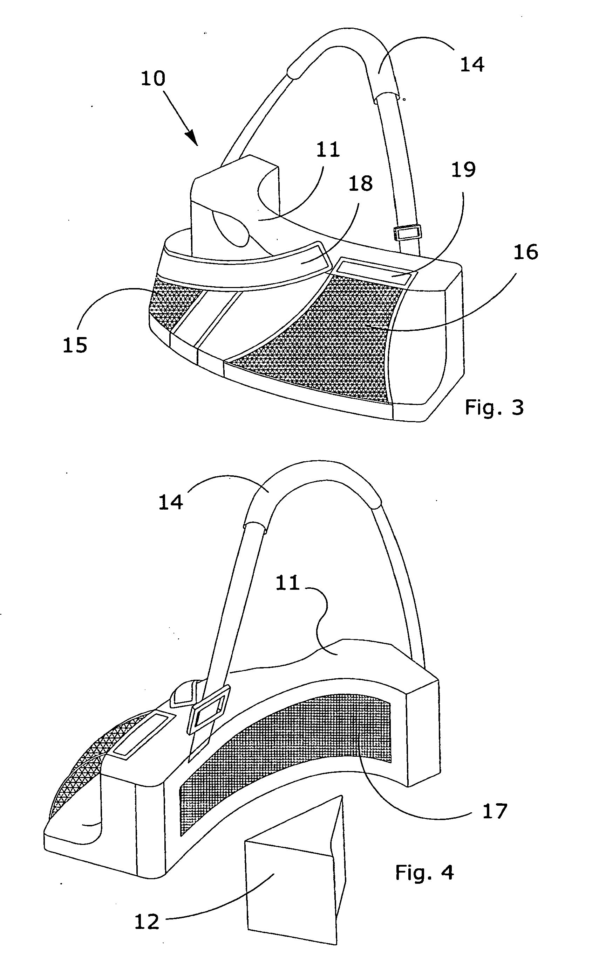

- figure 3 shows the antirotation orthopedic cushion according to the invention with both fastening flaps in the closed position;
- figure 4 is a view of the antirotation orthopedic cushion according to the invention with the wedge element detached from the body of the cushion;
- figure 5 is a view of the antirotation orthopedic cushion according to the invention with the wedge element attached to the body of the cushion;
- figure 6 is a schematic view of the complete fully assembled antirotation orthopedic cushion according to the invention;
- figure 7 is a schematic view of the antirotation orthopedic cushion according to the invention while being used by a patient in the extrarotation position;
- figures 8, 8' and 8'' are schematic views showing the buckle restraining device in the assembled condition, the buckle only and the support only, respectively;
- figure 9 is a schematic view showing the Velcro fastening system for attachment to the waist.

### DESCRIPTION OF ONE EMBODIMENT OF THE INVENTION

The antirotation orthopedic cushion according to the invention, indicated overall with the reference number 10, is an orthopedic device for immobilisation of upper limbs which have undergone reconstruction of the rotator cuffs.

According to the invention, the antirotation orthopedic cushion consists of three main elements:
A. a cushion 11 made from covered foam to be positioned at the level of the elbow joint between the arm and the trunk,
B. a wedge 12, also made from foam material, to be positioned between the cushion and the patient's waist;
C. a "restraining" system which consists of a belt 13 to be fastened around the waist, a shoulder strap 14, which is also designed to hold the cushion in the correct position, and two arm restraining flaps 15 and 16 made from transpirable material in order to ensure maximum comfort.

The cushion 11 has the feature of being shaped in such a way that:
a. it guarantees the non-retroposition of the shoulder even when the patient wears the device in bed; in fact the inner surface is designed in such a way that it is partly positioned in the lumbar area and thus prevents the aforesaid movement;
b. it provides a support area for the forearm which has the dual advantage of guaranteeing considerable comfort and, consequently of ensuring the correct shoulder muscle relaxation.

The wedge 12, also made from foam material, is positioned between the cushion and the patient's waist, and is held firmly in the required position thanks to two strips of Velcro sewn onto its padding which fix it to the Velcro 17 on the inner side of the cushion 11 and to the belt.

The wedge 12 makes it possible to determine the angle of intra or extra - rotation at which the joint must be blocked, and this is achieved by simply moving the wedge "forward", with respect to the position in which the joint is in a neutral condition (0°), for intrarotation and vice versa for extrarotation.

The device restraining system consists of the belt 13 to be fastened around the waist which holds the cushion in place by means of the two Velcro straps, and the shoulder strap 14 which also holds the cushion in the correct position.

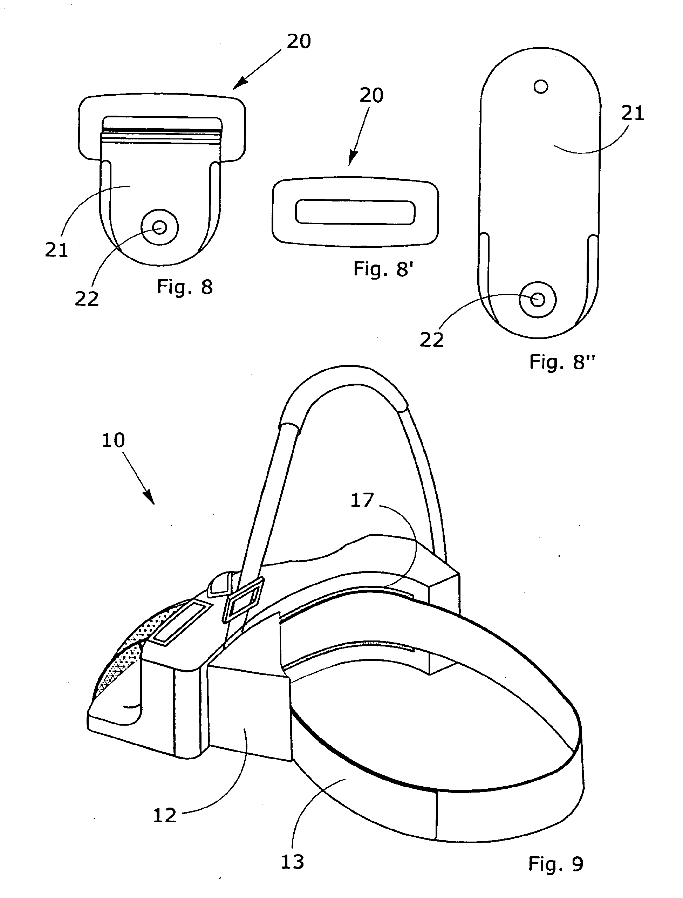

The shoulder strap 14 can be adjusted in length and position by means of a system fixing it to the cushion with an adjustable buckle 20 shown in figure 8, fitted on a support 21 equipped with respective restraining means that can be attached to the fixing points 22, which allow it to tilt.

This allows correct adaptation to the anatomical shape of the patient, thus favouring the stability of the device and comfort.

The shoulder strap is made in such a way that it is always the perfect length, and the double quick fastening Velcro system allows the shoulder strap to be unfastened without altering the set length.

Finally, the two arm restraining flaps 15 and 16 are made from transpirable material in order to ensure maximum comfort.

They consist of two flaps of material with strips of Velcro 18 and 19 at their ends which can be attached to the respective Velcro strips on the cushion 11.

They are designed to maintain the arm and thus the shoulder in the correct position.

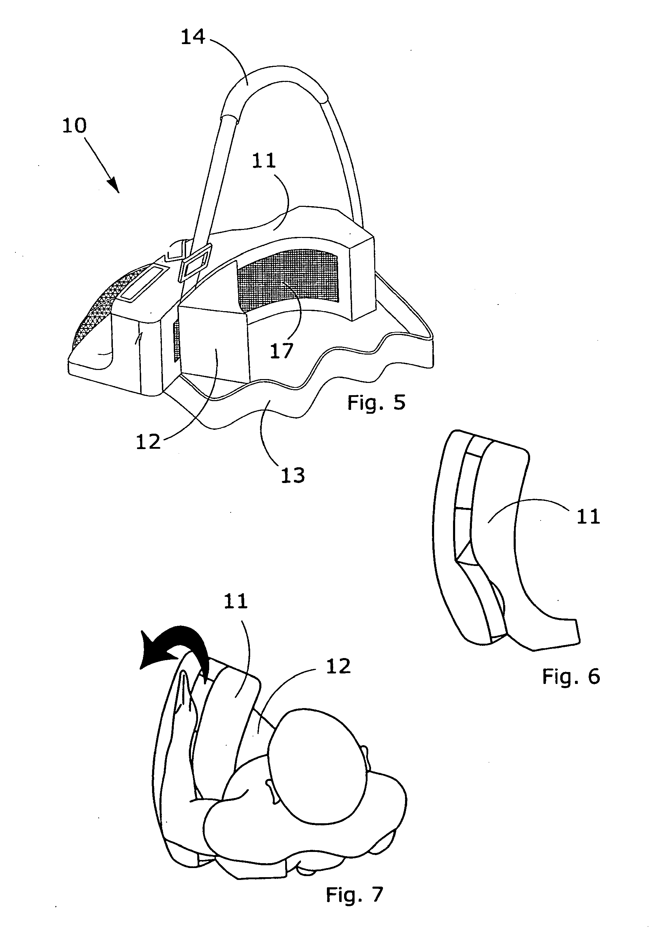

Figures 6 and 7 show the device according to the invention and how it is fitted on a patient.

As mentioned above, the device according to the invention makes it possible to prevent the retroposition of the scapula-thoracic joint.

The surface obtained by extending the profile of the cushion in a posterior direction with respect to the sagittal plane of the body, i.e. towards the lumbar area, creates a support that makes it impossible for the wearer to make the aforesaid movement, thus preventing problems, for example in the post-operative period (as in the case of protecting newly formed structures or stitches, etc.).

Adopting a solution of this type is also useful when the patient must wear the device at night when not being awake implies less movement control, while this function was previously entrusted to an optional strap extending from the pad on the contralateral shoulder to the shoulder supported by the cushion in an anteroposterior direction.

It should also be remembered that the wedge 12 has a dual function: on one hand it ensures that the device can be adapted to various anatomical shapes thus reducing production costs (one size fits all) without affecting the functionality of the device and on the other hand it has a therapeutic function, since it allows the health care operator to block the patient's joint not only in an neutral position but also in extrarotation or intrarotation, as can be seen in figure 7.

The advantage of the device according to the invention is essentially due to its versatility, and it is in fact sufficient to merely alter the position of the wedge 12 to obtain the desired angles.

The solution according to the invention also makes it possible to alter the shape of the cushion and more specifically the area in contact with the body, always maintaining the best possible contact with the patient's trunk and thus providing a high degree of comfort as well as always ensuring the correct action of the device.

The support surface for the forearm is designed to provide the dual advantage of guaranteeing comfort as it supports the weight of the arm and, consequently, keeping the shoulder muscles relaxed.

This support surface, together with the system of straps and transpirable fabrics, is designed to enclose the forearm, which in a competitor's product is represented by a pocket, with the undoubted advantage of being able to adjust the position of the straps to better adapt to the patient.

The difference between the two products relative to the strap fastening system must also be taken into consideration; in fact in the case of the solution according to the invention the straps are considerably simplified and less cumbersome thus increasing the level of comfort. In addition, with respect to competitors' products an adjustable buckle fastening system, shown in figure 8, has been added, allowing the system to adapt better to the patient's anatomical shape.

The strap-fastening adjustable buckle system according to the invention consists of a buckle 20, which is held by a buckle-holder 21 which, in turn, can be mounted on the cushion with a snap fastener system, in particular on the rear part of the cushion. This allows the buckle to rotate with respect to the axis of the snap fastener and, consequently, the position of the shoulder strap to be adjusted.

The invention is described above with reference to a preferred embodiment.

## Claims

1. An antirotation orthopedic cushion (10) in particular for the post-traumatic and post-surgical treatment of the shoulder, comprising a body (11) made from covered foam, to be positioned at the level of the elbow joint between the arm and the trunk, this body (11) being used in conjunction with a wedge (12), also made from foam material, and a "restraining" system consisting of a belt (13) to be fastened around the waist, and a shoulder strap (14), **characterised in that** said body (11) comprises a surface to be partly positioned in the lumbar area of a user in order to prevent the retroposition of the scapula-thoracic joint, **in that** said wedge (12) is positioned between the cushion and the patient's waist, and is held firmly in the required position thanks to two strips of Velcro sewn onto its padding which fix it to the Velcro (17) on the inner side of the cushion (11) and to the belt, and **in that** it further comprises two arm restraining flaps (15, 16) made from transpirable material in order to ensure maximum comfort.

2. An antirotation orthopedic cushion (10) according to said body (11) comprises a support area for the user's forearm, cooperating with said arm restraining flaps (15, 16).

3. A cushion (10) according to any one of the preceding claims, **characterised in that** the shoulder strap (14) can be adjusted in length and position by means of a system fixing it to the cushion with an adjustable buckle (20), which is held by a buckle-holder (21) which, in turn, can be mounted on the cushion with a snap fastener system at a point (22) located on the rear part of the cushion.

## Patentansprüche

1. Drehfestes orthopädisches Kissen (10) insbesondere für die posttraumatische und postchirurgische Behandlung der Schulter, bestehend aus einem aus bezogenem Schaumstoff hergestellten Körper (11), der auf Höhe des Ellbogengelenks zwischen Arm und Rumpf anzubringen ist, wobei dieser Körper (11) in Verbindung mit einem ebenfalls aus Schaumstoff gefertigten Keil (12) und einem aus einem um die Taille zu befestigenden Gürtel (13) und einem Schulterriemen (14) bestehenden Haltesystem zu benutzen ist, **dadurch gekennzeichnet, dass** der Körper (11) eine Oberfläche aufweist, die teilweise in der Lendengegend eines Trägers ruht, um die Retroposition des skapulothorakalen Nebengelenks zu verhindern, dass der Keil (12) zwischen dem Kissen und der Taille des Patienten angebracht ist und durch den Gürtel sowie durch zwei in seine Polsterung eingenähte Velcrostreifen fest in der erforderlichen Stellung gehalten wird, die ihn am Velcro (17) auf der Innenseite des Kissens (11) festhalten, und dass er weiterhin aus zwei den Arm umfangenden Laschen (15, 16) aus atmungsaktivem Material besteht, die ein maximal bequemes Tragen ermöglichen.

2. Drehfestes orthopädisches Kissen (10 gemäß dem besagten Körper (11), bestehend aus einem Stützbereich für den Unterarm des Trägers, der mit den Haltelaschen (15, 16) zusammenwirkt.

3. Kissen (10) gemäß einem der vorhergehenden Ansprüche, **dadurch gekennzeichnet, dass** der Schulterriemen (14) in Länge und Position mit einem System verstellt werden kann, das ihn am Kissen mit einer verstellbaren Schnalle (20) befestigt, die von einem Schnallenhalter (21) gehalten wird, der am Kissen mit einem Druckknopfsystem an einem Punkt (22) aufgesetzt ist, der sich im hinteren Teil des Kissens befindet.

## Revendications

1. Coussin orthopédique anti-rotation (10) notamment pour le traitement post-traumatique ou post-chirurgical de l'épaule, ledit coussin comportant un corps (11) réalisé à partir d'une mousse dotée d'un revêtement, destiné à être positionné au niveau de l'articulation du coude entre le bras et le tronc, ce corps (11) étant utilisé conjointement avec un élément cunéiforme (12), également réalisé à partir d'un matériau alvéolaire, et un système de « contention » consistant en une ceinture (13) destinée à être fixée autour de la taille, et une sangle d'épaule (14), **caractérisé en ce que** ledit corps (11) présente une surface destinée à être positionnée en partie dans la région lombaire d'un utilisateur afin d'empêcher l'articulation scapulo-thoracique de se déplacer en arrière, **en ce que** ledit élément cunéiforme (12) est positionné entre le coussin et la taille du patient, et est maintenu fermement dans la position requise grâce aux deux bandes velcros cousues sur son rembourrage qui le fixe à la bande velcro (17) du côté intérieur du coussin (11) et à la ceinture, et **en ce qu'**il comprend en outre deux pattes de maintien de bras (15, 16) réalisées à partir d'un matériau respirable afin de garantir un confort maximum.

2. Coussin orthopédique anti-rotation (10) selon la revendication 1, **caractérisé en ce que** ledit corps (11) comprend une zone de support destinée à l'avant-bras de l'utilisateur, coopérant avec lesdites pattes de maintien de bras (15, 16).

3. Coussin (10) selon l'une quelconque des revendications précédentes, **caractérisé en ce que** la sangle d'épaule (14) peut être réglée en longueur et en position au moyen d'un système qui la fixe au coussin au moyen d'une boucle réglable (20), laquelle est maintenue par un porte-boucle (21) qui peut être monté à son tour sur le coussin au moyen d'un système de fixation par encliquetage en un point (22) situé sur la partie arrière du coussin.
